# EUROPEAN PATENT APPLICATION

(11) **EP 3 903 719 A1**
(43) Date of publication of application: **03.11.2021**
(21) Application number: 19905155.8
(22) Date of filing: 24.12.2019
(51) Int. Cl.: A61B 34/35, A61B 50/13

(54) **MEDICAL CART**

(30) Priority: 26.12.2018 JP 2018243425
(71) Applicant: Kawasaki Jukogyo Kabushiki Kaisha, Hyogo 650-8670 (JP); Medicaroid Corporation, Hyogo 650-0047 (JP)
(72) Inventor: FUKUNO, Tomohiro, Hyogo, 650-8670 (JP); DOI, Wataru, Hyogo, 650-8670 (JP)
(74) Representative: Müller Hoffmann & Partner
(86) International application number: PCT/JP2019/050509
(87) International publication number: WO 2020/138032

(57) **Abstract**

There is provided a medical cart which has no risk of oil leakage and can quickly release fixing realized by stabilizers in an emergency. The medical cart includes: a base holding a positioner configured to move a multiple degree-of-freedom manipulator arm including a tip end portion holding a medical device; a base mount holding the base; a plurality of wheels including a pair of front wheels and a pair of rear wheels which move the base mount; and a plurality of stabilizers provided at the base mount and including respective ground contact portions which contact a ground surface when the stabilizers are extended by pneumatic pressure.

## Description

### Technical Field

The present invention relates to a medical cart configured to move a positioner configured to move a multiple degree-of-freedom manipulator arm holding a medical device.

### Background Art

Conventionally known is a medical cart which includes one or a plurality of manipulator arms and performs a surgical operation or predetermined work by moving the manipulator arms based on manipulation of an operator. Such medical cart includes stabilizers configured to fix the cart during the surgical operation or the work.

For example, PTL 1 discloses a neurological-surgery multifunctional robot platform which includes a position determining robot arm and moves legs by hydraulic jacks to fix a cart that is movable by casters.

PTL 2 discloses a medical robot system including a plurality of stabilizer assemblies. Each of the stabilizer assemblies includes: a feed screw rotated by a motor; and a stabilization leg attached to a nut which moves up and down by the rotation of the feed screw.

### Citation List

### Patent Literature

PTL 1: Published Japanese Translation of PCT Application No. 2010-530268
PTL 2: U.S. Patent Application Publication No. 2017/0101118

### Summary of Invention

### Technical Problem

However, according to the cart of PTL 1, since the legs (stabilizers) are moved by the hydraulic jacks connected to a hydraulic circuit, the risk of oil leakage is unavoidable. Moreover, according to the medical robot system of PTL 2, there is a problem that the fixing of a medical device by the stabilization legs cannot be quickly released in an emergency. Especially when power supply is cut off, it is difficult to quickly release the fixing realized by the plurality of stabilization legs.

An object of the present invention is to provide a medical cart which has no risk of oil leakage and can quickly release fixing realized by stabilizers in an emergency.

### Solution to Problem

A medical cart of the present invention includes: a base holding a positioner configured to move a multiple degree-of-freedom manipulator arm including a tip end portion holding a medical device; a base mount holding the base; a plurality of wheels including a pair of front wheels and a pair of rear wheels which move the base mount; and a plurality of stabilizers provided at the base mount and including respective ground contact portions which contact a ground surface when the stabilizers are extended by pneumatic pressure.

According to the present invention, since the stabilizers are extended or contracted by pneumatic pressure, the stabilizers have no risk of oil leakage unlike stabilizers which are extended or contracted by hydraulic pressure, and therefore, are preferably used in surgical sites. Moreover, only by releasing the pneumatic pressure which holds the extended states of the stabilizers, the stabilizers can be made to quickly contract in an emergency, and the ground contact portions can be separated from a surface, such as an interior floor.

In the above invention, it is preferable that the ground contact portions of the stabilizers contact the ground surface in a state where the wheels do not float from the ground surface.

According to the above configuration, when removing air from the stabilizers, i.e., when making the stabilizers contract, impact applied to the medical cart since the wheels are not in contact with the ground surface can be prevented.

In the above invention, the plurality of stabilizers may include two front stabilizer arranged at a front portion of the base mount and two rear stabilizers arranged at a rear portion of the base mount.

According to the above configuration, the medical cart can be stably supported in a state where the two front stabilizers are extended, and the two rear stabilizers are extended.

In the above invention, the pair of front wheels may include a left front wheel and a right front wheel arranged so as to be spaced apart from each other in an orthogonal direction that is a direction orthogonal to a proceeding direction. The rear wheels may include a left rear wheel and a right rear wheel arranged so as to be spaced apart from each other in the orthogonal direction. The two front stabilizers may be arranged between the left front wheel and the right front wheel in the orthogonal direction. One of the rear stabilizers may be arranged outside the left rear wheel in the orthogonal direction. The other of the rear stabilizers may be arranged outside the right rear wheel in the orthogonal direction.

According to the above configuration, since the two front stabilizers are arranged between the left front wheel and the right front wheel in the above orthogonal direction, the front stabilizers do not interfere with the rotating operations of the wheels as compared to when one of the front stabilizers is arranged outside the left front wheel in the same direction, and the other of the front stabilizers is arranged outside the right front wheel in the same direction.

Moreover, one of the rear stabilizers is arranged outside the left rear wheel in the above orthogonal direction, and the other of the rear stabilizers is arranged outside the right rear wheel in the same direction. With this, a small interval between the left rear wheel and the right rear wheel can be designed. Thus, when a lever held by an operator is provided above the rear wheels, the operator can change the proceeding direction of the medical cart by small force because of the above small interval between the rear wheels.

In the above invention, the medical cart may further include a steering portion including a steering shaft and a handle provided at the steering shaft, the steering portion being arranged at a rear side of the base mount in a proceeding direction, the steering shaft being used to change directions of the pair of rear wheels. An interval between the pair of rear wheels may be smaller than an interval between the pair of front wheels.

According to the above configuration, since an interval between the pair of rear wheels is set to be smaller than an interval between the pair of front wheels, operability when changing the directions of the pair of rear wheels by the handle can be improved.

In the above invention, the pair of front wheels may be driving wheels. A pair of auxiliary wheels may be provided between the pair of front wheels and the pair of rear wheels. An interval between the pair of auxiliary wheels may be larger than an interval between the pair of rear wheels.

According to the above configuration, the directions of the pair of rear wheels can be easily changed by the existence of the auxiliary wheels.

In the above invention, the pair of front wheels may be driving wheels. The medical cart may further include: brake devices respectively provided at the pair of front wheels; and a brake releasing tool configured to release braking of the brake devices in an emergency.

According to the above configuration, in an emergency which requires the release of the braking of the brake devices, such release can be easily and quickly performed by the brake releasing tool.

In the above invention, each of the plurality of stabilizers may include an air cylinder including a piston rod configured to advance or retreat and the ground contact portion provided at a tip end portion of the piston rod.

According to the above configuration, the piston rods can be easily extended by the air cylinders, and with this, the ground contact portions can be made to contact the ground surface.

In the above invention, the air cylinders of the plurality of stabilizers may be pneumatic lock air cylinders.

According to the above configuration, supply and discharge of the air of the air cylinders can be locked. With this, the extended states of the stabilizers can be held (locked).

In the above invention, each of the plurality of air cylinders may include a biasing member configured to bias the piston rod in such a direction that the piston rod contracts.

According to the above configuration, when releasing the air in the air cylinders of the stabilizers, the piston rods can be made to contract by the biasing force of the biasing members, and therefore, the ground contact states of the stabilizers can be released.

In the above invention, the medical cart may further include a compressed air supply source configured to supply compressed air to the air cylinders of the plurality of stabilizers.

According to the above configuration, the compressed air can be supplied to a plurality of air cylinders by one compressed air supply source.

In the above invention, the medical cart may further include: an electromagnetic valve configured to be switchable between a state where the compressed air is supplied from the compressed air supply source to the plurality of air cylinders and a state where the compressed air is not supplied from the compressed air supply source to the plurality of air cylinders; and a manual valve arranged between the electromagnetic valve and the plurality of air cylinders and configured to discharge the compressed air to an atmosphere.

According to the above configuration, in an emergency, such as when power supply is cut off, the air in the air cylinders of the stabilizers can be released by the manual valve. With this, the stabilizers can be made to contract by a manual method, and the ground contact states of the stabilizers can be released. Thus, the medical cart can be set to a movable state even when power supply is cut off.

In the above invention, the medical cart may further include a manual operation tool configured to operate both the brake releasing tool and the manual valve.

According to the above configuration, in an emergency, such as when power supply is cut off, both the brake releasing tool and the manual valve can be easily operated by the manual operation tool. With this, the braking of the brake devices can be released, and the ground contact states of the stabilizers can also be released.

In the above invention, the medical cart may further include an input device configured to receive instructions of ground contact operations and ground contact releasing operations of the plurality of stabilizers from an operator.

According to the above configuration, the operator can easily instruct the ground contact operations and the ground contact releasing operations of the stabilizers by using the input device.

### Advantageous Effects of Invention

The present invention can provide the medical cart which has no risk of oil leakage and can quickly release the fixing realized by the stabilizers in an emergency.

### Brief Description of Drawings

FIG. 1 is a diagram showing a surgical operation system including a medical cart according to one embodiment of the present invention.
FIG. 2 is a diagram showing the configuration of a manipulator arm of a medical manipulator shown in FIG. 1.
FIG. 3 is a block diagram showing the configuration of a control system in the medical manipulator shown in FIG. 1.
FIG. 4 is a perspective view showing the medical cart according to one embodiment of the present invention.
FIG. 5 is a perspective view showing the medical cart from which a casing shown in FIG. 4 is detached.
FIG. 6 is a block diagram showing the configuration of a control system in the medical cart.
FIG. 7 is a side view showing the medical cart shown in FIG. 4.
FIG. 8 is a bottom view showing the medical cart shown in FIG. 4.
FIG. 9 is a perspective view showing an input device.
FIG. 10A is a sectional view for explaining braking of a brake device. FIG. 10B is a sectional view for explaining release of the braking of the brake device.
FIG. 11 is a perspective view showing a brake releasing tool.
FIG. 12 is a diagram showing a pneumatic circuit configured to realize expansion and contraction of a stabilizer.

### Description of Embodiments

Hereinafter, a medical cart according to one embodiment of the present invention will be described with reference to the drawings. The medical cart described below is merely one embodiment of the present invention. Therefore, the present invention is not limited to the following embodiment, and additions, deletions, and modifications may be made within the scope of the present invention.

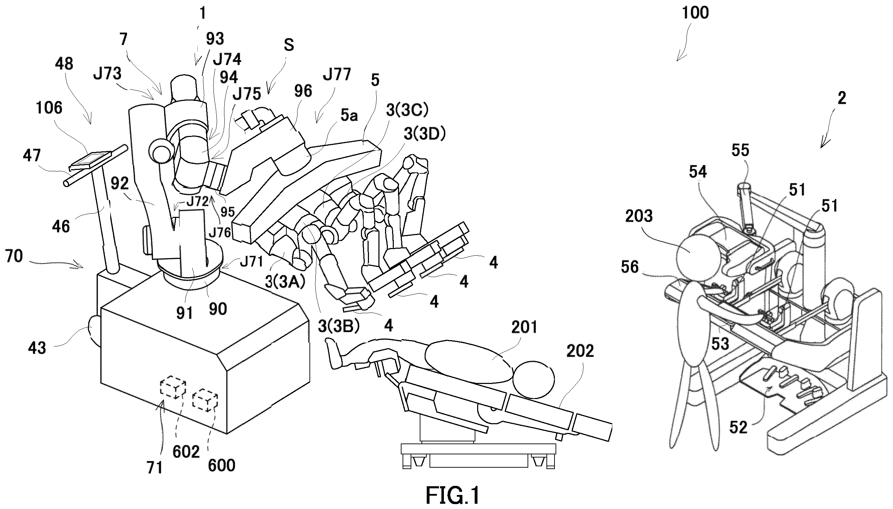

As shown in FIG. 1, a medical cart 70 of the present embodiment is provided at a surgical operation system 100 that is a system used when performing an endoscope surgical operation for a patient 201 on an operating table 202 in, for example, a robot assisted operation or a robot remote operation. First, the entire surgical operation system 100 will be described, and then, the medical cart 70 according to the present embodiment will be described.

The surgical operation system 100 includes: a medical manipulator 1 that is a patient-side system; and an instructing apparatus 2 used to manipulate the medical manipulator 1. The instructing apparatus 2 is arranged away from the medical manipulator 1, and the medical manipulator 1 is remotely manipulated by the instructing apparatus 2. An operator 203 who is a surgeon inputs to the instructing apparatus 2 an operation to be performed by the medical manipulator 1, and the instructing apparatus 2 transmits an operating command corresponding to the input operation to the medical manipulator 1. The medical manipulator 1 receives the operating command transmitted from the instructing apparatus 2 and operates long shaft-shaped medical devices 4, such as endoscope assemblies and/or instruments, included in the medical manipulator 1 based on the operating command.

The instructing apparatus 2 constitutes an interface between the surgical operation system 100 and the operator 203 and is an apparatus used to manipulate the medical manipulator 1. The instructing apparatus 2 is arranged inside or outside an operating room. The instructing apparatus 2 includes: a manipulator arm 51, a manipulation pedal 52, and a touch panel 53 by which the operator 203 inputs the operating command; a monitor 54 configured to display an image taken by the endoscope assembly; a support arm 55 supporting the monitor 54 at a height position corresponding to the face of the operator 203; a bar 56 at which the touch panel 53 is arranged; and the like. While visually confirming an affected part on the monitor 54, the operator 203 manipulates the manipulator arm 51 and the manipulation pedal 52 to input the operating command to the instructing apparatus 2. The operating command input to the instructing apparatus 2 is transmitted to a controller 600 of the medical manipulator 1 through wired communication or wireless communication. The operation of the medical manipulator 1 is controlled by the controller 600. It should be noted that the controller 600 is constituted by a computer, such as a microcontroller. The controller 600 and a storage portion 602 are accommodated in the medical cart 70. The storage portion 602 stores control programs and various data used for the control of the operation. Moreover, the medical cart 70 is provided with an input device 106 manipulated to move the positions of or change the postures of a positioner 7, an arm base 5, and a plurality of arms 3 mainly before surgery.

The medical manipulator 1 constitutes an interface between the surgical operation system 100 and the patient. The medical manipulator 1 is arranged in the operating room that is a sterile field.

In FIG. 1, the medical manipulator 1 includes: the positioner 7; the long arm base 5 attached to a tip end portion of the positioner 7; and a plurality of (four in the present embodiment) multiple degree-of-freedom manipulator arms (hereinafter simply referred to as "arms") 3 each including a base end portion detachably attached to the arm base 5. The medical manipulator 1 is configured such that the plurality of arms 3 are folded, i.e., take storage postures.

The positioner 7 is configured as a vertical articulated robot. The positioner 7 is provided at a base 20 arranged on a casing 71 of the medical cart 70 arranged at a predetermined position in the operating room. The positioner 7 can three-dimensionally move the position of the arm base 5. The arms 3 and the arm base 5 are covered with a sterile drape (not shown) and to be shielded from the sterile field in the operating room.

The positioner 7 includes: a base 90 attached to a cart main body 71; and a plurality of positioner link portions which are sequentially coupled to each other from the base 90 toward the tip end portion. The positioner 7 constitutes a plurality of joint portions by sequentially coupling the positioner link portions to each other such that one positioner link portion rotates relative to another positioner link portion. The plurality of positioner link portions include first to sixth links 91 to 96. The plurality of joint portions include first to seventh joints J71 to J77. It should be noted that each of the plurality of joint portions in the present embodiment is constituted by a rotational joint including a rotating shaft. However, at least some of the joint portions may be constituted by linear motion joints.

More specifically, a base end portion of the first link 91 is coupled to a tip end portion of the base 90 through the first joint J71 that is a twisting (roll) j oint. A base end portion of the second link 92 is coupled to a tip end portion of the first link 91 through the second joint J72 that is a bending (pitch) joint. Abase end portion of the third link 93 is coupled to a tip end portion of the second link 92 through the third joint J73 that is a bending joint. A base end portion of the fourth link 94 is coupled to a tip end portion of the third link 93 through the fourth joint J74 that is a twisting joint. A base end portion of the fifth link 95 is coupled to a tip end portion of the fourth link 94 through the fifth joint J75 that is a bending joint. A base end portion of the sixth link 96 is coupled to a tip end portion of the fifth link 95 through the sixth joint J76 that is a twisting joint. A positioner attaching portion 5a of the arm base 5 is coupled to a tip end portion of the sixth link 96 through the seventh joint J77 that is a twisting joint. With this, the positioner 7 is configured as a multiaxial joint (seven-axis joint) arm having plural degrees of freedom (seven degrees of freedom).

For example, a spare instrument (such as a pair of forceps) as the medical device 4 is held by a tip end portion of an arm 3A among the plurality of arms 3. An instrument, such as a pair of forceps, as the medical device 4 is held by a tip end portion of an arm 3B among the plurality of arms 3. For example, an endoscope assembly as the medical device 4 is held by a tip end portion of an arm 3C among the plurality of arms 3. For example, a spare endoscope assembly as the medical device 4 is held by a tip end portion of an arm 3D among the plurality of arms 3.

In the medical manipulator 1, the arm base 5 serves as a hub for the plurality of arms 3. In the present embodiment, the positioner 7 and the arm base 5 constitute a manipulator arm support body S supporting the plurality of arms 3 such that the plurality of arms 3 are movable.

In the medical manipulator 1, components from the positioner 7 to the medical device 4 are coupled to each other in series. Hereinafter, in the present description, regarding each of these components, an end portion located closer to the positioner 7 is referred to as a "base end portion," and an opposite end portion is referred to as a "tip end portion."

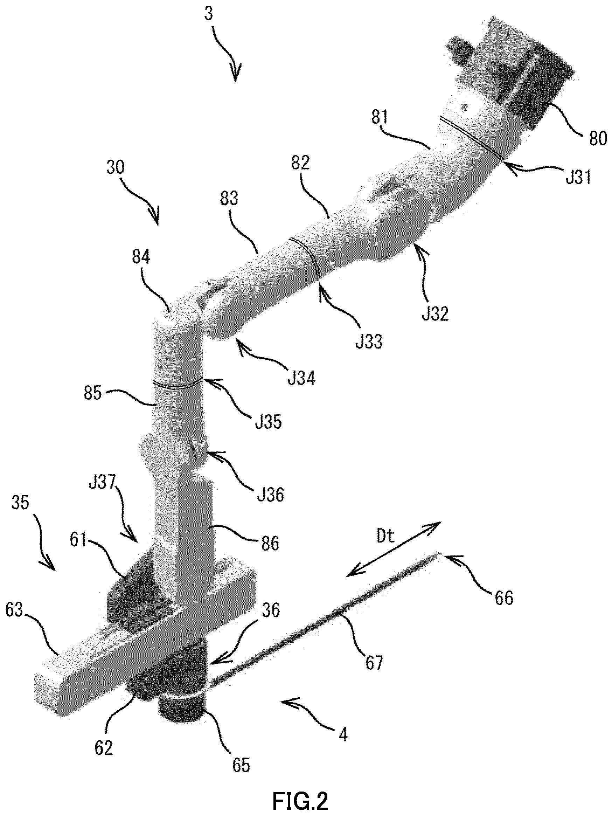

As shown in FIG. 2, when the medical device 4 is an instrument, the medical device 4 includes a drive unit 65 provided at the base end portion thereof. An end effector provided at the tip end portion of the instrument is selected from the group consisting of tools (such as a pair of forceps, a pair of scissors, a grasper, a needle holder, a microdissector, a staple applier, a tucker, a suction cleaning tool, a snare wire, and a clip applier) each having an operating joint and tools (such as a cutting blade, a cautery probe, a washer, a catheter, a suction orifice) each having a joint.

In the surgery using the medical manipulator 1, first, the medical cart 70 is moved to a predetermined position in the operating room by a medical worker, such as an assistant or a nurse. In this case, the medical cart 70 which has been moved to the predetermined position is stopped by a below-described configuration so as not to move to an unexpected position. Details will be described later.

Next, the medical worker manipulates a touch panel included in the input device 106 to operate the positioner 7. With this, the arm base 5 is positioned such that a predetermined positional relation between the arm base 5 and the operating table 202 or between the arm base 5 and the patient 201 is realized. Next, the controller 600 operates each arm 3 to position the medical device 4 such that a predetermined initial positional relation between the medical device 4 and a sleeve (cannula sleeve) placed on a body surface of the patient 201 is realized. It should be noted that the positioning operation of the positioner 7 and the positioning operation of each arm 3 may be performed at the same time. Then, with the positioner 7 stopped basically, the controller 600 performs the surgery by operating the medical device 4 by each arm 3 in accordance with the operating command from the instructing apparatus 2 while suitably changing the position and posture of the medical device 4.

Next, the detailed configuration of the arm 3 will be described. As shown in FIG. 2, each arm 3 includes an arm main body 30 and a translational unit 35 coupled to the tip end portion of the arm main body 30 and is configured such that the tip end portion thereof is movable relative to the base end portion thereof in a three-dimensional space. It should be noted that in the present embodiment, the plurality of arms 3 included in the medical manipulator 1 are the same in configuration as each other or are similar in configuration to each other. However, at least one of the plurality of arms 3 may be different in configuration from the other arms. A holder 36 which can hold the medical device 4 is provided at the tip end portion of the arm 3. The medical device 4 includes: the drive unit 65 provided at the base end portion thereof; an end effector (treatment tool) 66 provided at the tip end portion thereof; and a long and thin shaft 67 connecting the drive unit 65 and the end effector 66. The drive unit 65, the shaft 67, and the end effector 66 are arranged along a long-axis direction Dt. The holder 36 includes a servomotor M39 and is configured such that a rotating body provided at the drive unit 65 of the medical device 4 attached to the holder 36 is rotated by the servomotor M39. The drive unit 65 operates the end effector 66 by the rotation of the rotating body.

The arms 3 are configured to be attachable to and detachable from the arm base 5. The arms 3 have water resistance, heat resistance, and chemical resistance for a cleaning treatment and a sterilization treatment. There are various methods as the sterilization treatment of the arms 3. For example, high pressure steam sterilization, EOG sterilization, chemical sterilization using disinfectant, or the like is selectively used.

The arm main body 30 includes: a base 80 detachably attached to the arm base 5; and first to sixth links 81 to 86 sequentially coupled to each other from the base 80 toward the tip end portion. More specifically, the base end portion of the first link 81 is coupled to the tip end portion of the base 80 through a twisting joint J31. The base end portion of the second link 82 is coupled to the tip end portion of the first link 81 through a bending joint J32. The base end portion of the third link 83 is coupled to the tip end portion of the second link 82 through a twisting joint J33. The base end portion of the fourth link 84 is coupled to the tip end portion of the third link 83 through a bending joint J34. The base end portion of the fifth link 85 is coupled to the tip end portion of the fourth link 84 through a twisting joint J35. The base end portion of the sixth link 86 is coupled to the tip end portion of the fifth link 85 through a bending joint J36. The base end portion of the translational unit 35 is coupled to the tip end portion of the sixth link 86 through a bending joint J37. With this, each arm 3 is configured as a multiaxial joint (seven-axis joint) arm having plural degrees of freedom (seven degrees of freedom). Therefore, each arm 3 can change its posture without changing the position of the tip end portion of the arm 3.

An outer shell of the arm main body 30 is mainly formed by a member, such as stainless steel, having heat resistance and chemical resistance. Moreover, a seal (not shown) for obtaining water resistance is provided at a coupling portion between the links. The seal has heat resistance corresponding to high pressure steam sterilization and chemical resistance with respect to disinfectant. It should be noted that regarding the coupling portion between the links, an end portion of one of the links is inserted into an end portion of the other link, and the seal is arranged so as to fill a gap between the end portions of the links, and is therefore hidden in terms of appearance. With this, infiltration of water, chemical liquid, and steam through the gap between the seal and the link is suppressed.

The translational unit 35 makes the holder 36, attached to the tip end portion thereof, perform a translational movement in the long-axis direction Dt to make the medical device 4, attached to the holder 36, perform a translational movement in a direction in which the shaft 67 extends.

The translational unit 35 includes: a base end-side link 61 coupled to the tip end portion of the sixth link 86 of the arm main body 30 through the bending joint J37; a tip end-side link 62; a coupling link 63 configured to move between the base end-side link 61 and the tip end-side link 62 in conjunction with the base end-side link 61 and the tip end-side link 62; and an interlock mechanism (not shown). The bending joint J37 extends in a direction orthogonal to the long-axis direction Dt. Moreover, the servomotor M39 configured to rotate the rotating body provided at the drive unit 65 of the medical device 4 is included in the tip end portion of the translational unit 35, i.e., in the holder 36 of the tip end-side link 62. A driving source of the translational unit 35 is provided at the base end-side link 61. The coupling link 63 extends along the long-axis direction Dt. According to this configuration, the translational unit 35 can change the position of the medical device 4, held by the holder 36 provided at the tip end-side link 62, relative to the base end-side link 61 in the long-axis direction Dt in such a manner that the interlock mechanism changes relative positions of the base end-side link 61 and the coupling link 63 in the long-axis direction Dt and relative positions of the coupling link 63 and the tip end-side link 62 in the long-axis direction Dt.

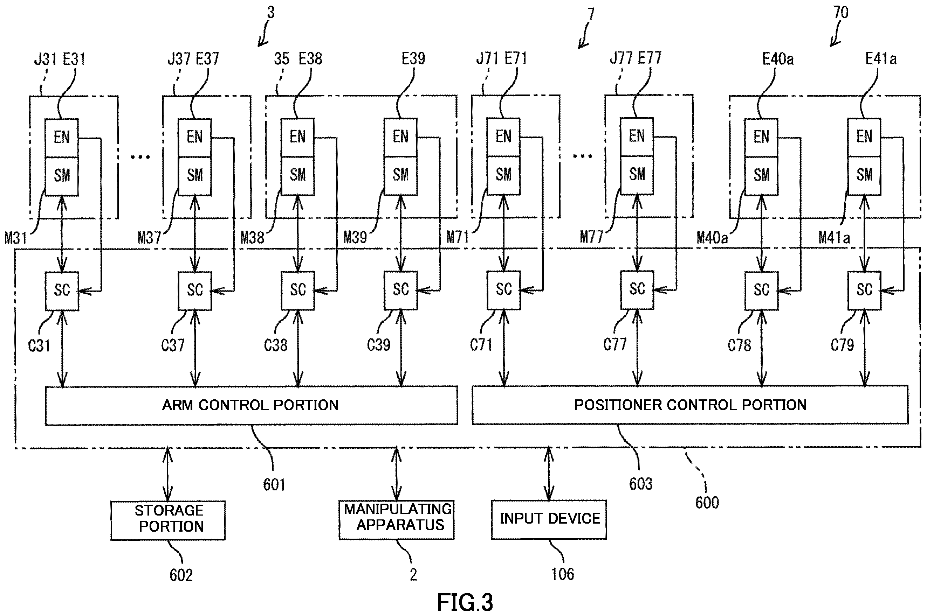

Next, as shown in FIG. 3, each arm 3 includes: driving servomotors M31 to M37 corresponding to the respective joints J31 to J37; encoders E31 to E37 corresponding to the respective joints J31 to J37 and configured to detect rotation angles of the respective servomotors M31 to M37; and speed reducers (not shown) corresponding to the respective joints J31 to J37 and configured to reduce the speeds of the outputs of the respective servomotors M31 to M37 to increase the torques of the outputs. It should be noted that in FIG. 3, among the joints J31 to J37, a control system of the twisting joint J31 and a control system of the bending joint J37 are representatively shown, and control systems of the other joints J33 to J36 are omitted. Moreover, the translational unit 35 includes: a servomotor M38 for a translational operation; the servomotor M39 configured to rotate the rotating body provided at the drive unit 65 of the medical device 4; encoders E38 and E39 configured to detect the rotation angles of the respective servomotors M38 and M39; speed reducers (not shown) configured to reduce the speeds of the outputs of the respective servomotors M38 and M39 to increase the torques of the outputs.

Moreover, as shown in FIG. 3, the positioner 7 includes: driving servomotors M71 to M77 corresponding to the respective joints J71 to J77 of the positioner 7; encoders E71 to E77 corresponding to the respective joints J71 to J77 of the positioner 7 and configured to detect the rotation angles of the respective servomotors M71 to M77; and speed reducers (not shown) corresponding to the respective joints J71 to J77 of the positioner 7 and configured to reduce the speeds of the outputs of the respective servomotors M71 to M77 to increase the torques of the outputs. It should be noted that in FIG. 3, among the joints J71 to J77 of the positioner 7, control systems of the joints J71 and J77 are representatively shown, and control systems of the other joints J72 to J76 are omitted. Moreover, as shown in FIG. 3, the medical cart 70 includes: driving servomotors M40a and M40b corresponding to respective front wheels 40 and 41; encoders E40a and E40b corresponding to the respective front wheels 40 and 41 and configured to detect the rotation angles of the respective servomotors M40a and M40b; and speed reducers (not shown) corresponding to the respective front wheels 40 and 41 and configured to reduce the speeds of the outputs of the respective servomotors M40a and M40b to increase the torques of the outputs.

The controller 600 includes an arm control portion 601 and a positioner control portion 603. The arm control portion 601 controls the movements of the plurality of arms 3 based on the operating command. The positioner control portion 603 controls the movement of the positioner 7 and the driving of the front wheels 40 and 41 of the medical cart 70. Servo control portions C31 to C37, C38, and C39 are electrically connected to the arm control portion 601. The encoders E31 to E37, E38, and E39 are electrically connected to the respective servo control portions C31 to C37, C38, and C39. Moreover, servo control portions C71 to C79 are electrically connected to the positioner control portion 603. The encoders E71 to E77, E40a, and E41a are electrically connected to the respective servo control portions C71 to C79.

Based on the operating command input to the instructing apparatus 2, a position posture command of the tip end portion of the arm 3 is input to the arm control portion 601. The arm control portion 601 generates a position command value based on the position posture command and the rotation angles detected by the encoders E31 to E37, E38, and E39 and outputs the position command value. The servo control portions C31 to C37, C38, and C39 which have acquired the position command value generate a driving command value (torque command value) based on the rotation angles detected by the encoders E31 to E37, E38, and E39 and the position command value and outputs the driving command value (torque command value). An amplifying circuit which has acquired the driving command value supplies a driving current corresponding to the driving command value to the servomotors M31 to M37, M38, and M39. Thus, the servomotors M31 to M37, M38, and M39 are servo-controlled such that the position and posture of the tip end portion of the arm 3 reach the position and posture corresponding to the position posture command.

The storage portion 602 from which the arm control portion 601 can read data is provided at the controller 600. The storage portion 602 prestores surgical information input through the instructing apparatus 2.

The positioner control portion 603 generates the position command value based on an operating command, input from the input device 106 and related to setting of a preparation position and the like, and the rotation angles detected by the encoders E71 to E77, and outputs the position command value. The servo control portions C71 to C77 which have acquired the position command value generate the driving command value (torque command value) based on the rotation angles detected by the encoders E71 to E77 and the position command value and output the driving command value (torque command value). The amplifying circuit which has acquired the driving command value supplies the driving current corresponding to the driving command value to the servomotors M71 to M77. Thus, the servomotors M71 to M77 are servo-controlled such that the position and posture of the positioner 7 reach the position and posture corresponding to the position command value.

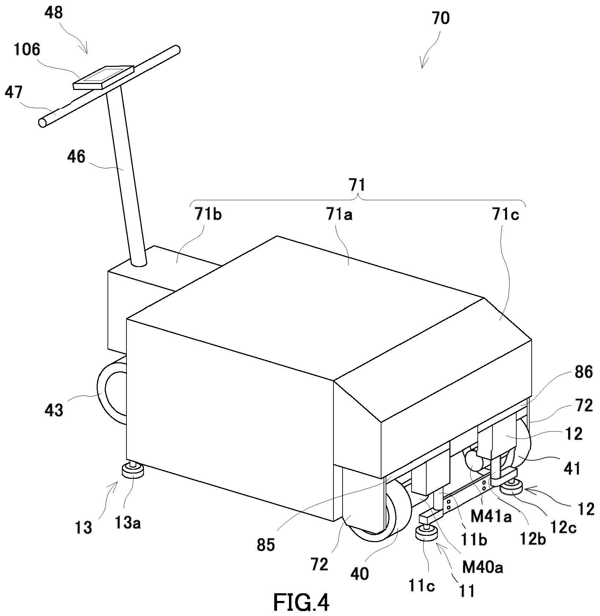

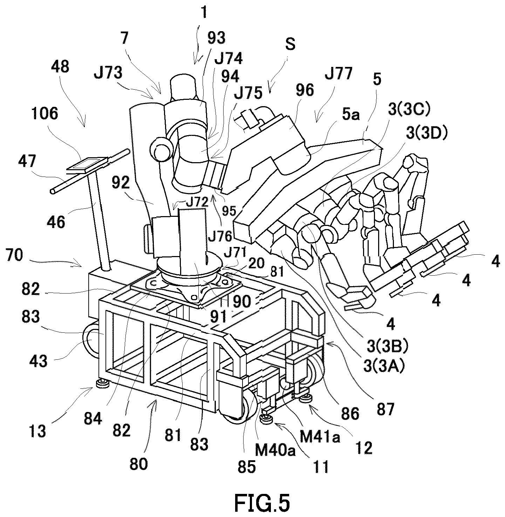

Next, the medical cart 70 of the present embodiment will be described. As shown in FIG. 4, the medical cart 70 includes the casing 71. Moreover, as shown in FIG. 5, the medical cart 70 includes a base mount (chassis) 80 at least part of which is provided in the casing 71. The casing 71 includes casing constituting portion 71a, 71b, and 71c. A bottom surface of the casing constituting portion 71a is located lower than bottom surfaces of the casing constituting portions 71b and 71c. It should be noted that the base 20 holding the positioner 7 of FIG. 1 is held by the base mount 80. It should be noted that in FIG. 4, the components from the positioner 7 to the medical device 4 are not shown.

The casing constituting portion 71b is connected to the casing constituting portion 71a and is arranged at a rear side of the casing constituting portion 71a (i.e., at a rear side in a proceeding direction of the medical cart 70; the same shall apply hereinafter). A width of the casing constituting portion 71b in a direction orthogonal to the proceeding direction is smaller than a width of the casing constituting portion 71a in the direction orthogonal to the proceeding direction. The casing constituting portion 71b is connected to a middle portion of the casing constituting portion 71a, the middle portion being located in the middle in the direction orthogonal to the proceeding direction. A below-described right rear wheel 43 and a below-described left rear wheel 44 are provided under the casing constituting portion 71b.

A steering portion 48 including a steering shaft 46 and a handle 47 is provided at the casing constituting portion 71b. The steering shaft 46 is provided in a standing state. The steering shaft 46 is a member configured to change the directions of the right rear wheel 43 and the left rear wheel 44. The handle 47 extending in a substantially horizontal direction is provided at the steering shaft 46. The medical worker can grasp the handle 47 and move the medical cart 70 to a predetermined position. Moreover, the medical worker can steer the medical cart 70 by the handle 47, and with this, can change the directions of the right rear wheel 43 and the left rear wheel 44. Thus, the proceeding direction of the medical cart 70 can be changed. Moreover, the input device 106 that is a user interface is provided in the vicinity of the handle 47. The input device 106 is constituted by, for example, a touch panel.

Moreover, a rotary grip (not shown) is provided at the handle 47. The medical worker can manipulate the rotary grip to move the medical cart 70 while adjusting the movement distance of the medical cart 70.

Moreover, the casing constituting portion 71c is connected to the casing constituting portion 71a and is arranged at a front side of the casing constituting portion 71a. A width of the casing constituting portion 71c in the direction orthogonal to the proceeding direction is equal to a width of the casing constituting portion 71a in the direction orthogonal to the proceeding direction. The casing constituting portion 71c is connected to the casing constituting portion 71a such that both side surfaces of the casing constituting portion 71c are respectively flush with both side surfaces of the casing constituting portion 71a. A below-described right front wheel 40 and a below-described left front wheel 41 are provided under the casing constituting portion 71c.

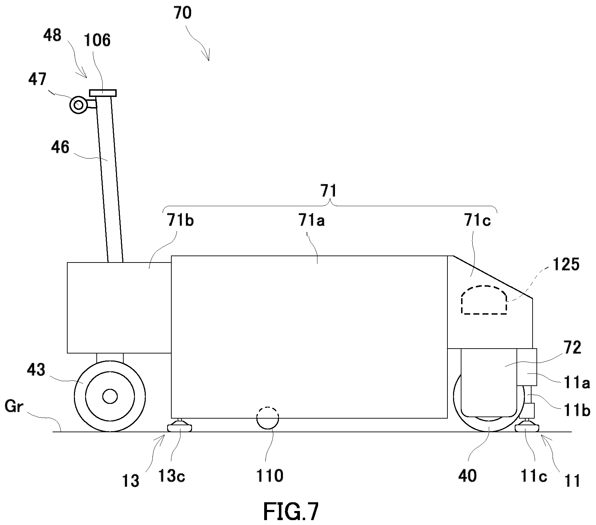

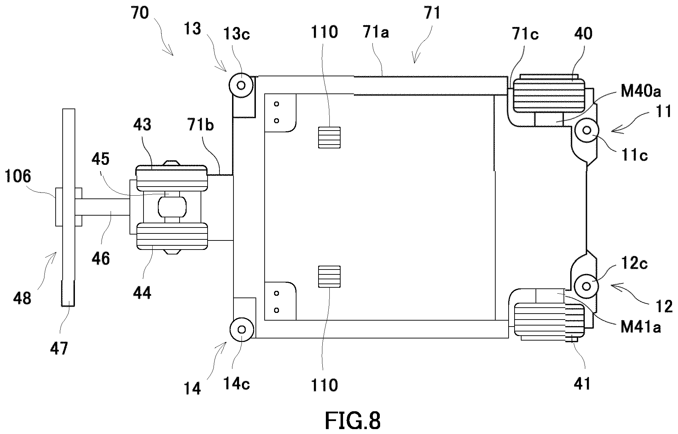

As shown in FIGS. 4 and 8, the medical cart 70 includes a plurality of wheels by which the casing 71 moves. Specifically, the medical cart 70 includes: the right front wheel 40 and the left front wheel 41 arranged so as to be spaced apart from each other in the direction orthogonal to the proceeding direction; and the right rear wheel 43 and the left rear wheel 44 arranged so as to be spaced apart from each other in the direction orthogonal to the proceeding direction. As shown in FIGS. 7 and 8, a pair of auxiliary wheels 110 are provided between a group of the right front wheel 40 and the left front wheel 41 and a group of the right rear wheel 43 and the left rear wheel 44 in a front-rear direction. An interval between the pair of auxiliary wheels 110 is set to be larger than an interval between the right rear wheel 43 and the left rear wheel 44. Moreover, plate-shaped cover members (covers) 72 supported by the bottom surface of the casing constituting portion 71c are provided outside the right front wheel 40 and outside the left front wheel 41, respectively. Hereinafter, the right front wheel 40, the left front wheel 41, the right rear wheel 43, and the left rear wheel 44 may be collectively called the wheels. It should be noted that although not shown, plate-shaped cover members (covers) supported by the bottom surface of the casing constituting portion 71c are also provided inside the right front wheel 40 and inside the left front wheel 41, respectively.

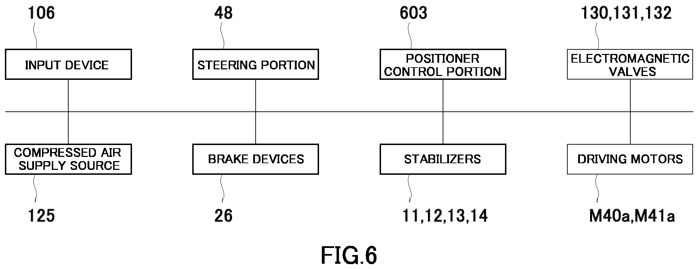

The driving servomotor M40a is connected to the right front wheel 40 through a driving shaft (not shown). Similarly, the driving servomotor M41a is connected to the left front wheel 41 through a driving shaft (not shown). The driving servomotors M40a and M41a are controlled by the positioner control portion 603 (see FIGS. 3 and 6), and with this, the right front wheel 40 and the left front wheel 41 are rotated. Moreover, the right rear wheel 43 and the left rear wheel 44 are coupled to each other through a connecting shaft 45. The right rear wheel 43 and the left rear wheel 44 are rotated based on the manipulation of the steering portion 48 by the medical worker. With this, the medical cart 70 moves.

As shown in FIG. 5, the base mount 80 of the medical cart 70 is configured as a frame structure. Specifically, the base mount 80 includes a pair of longitudinal frame members 81 and a plurality of transverse frame members 82. The pair of longitudinal frame members 81 extend in a front-rear direction (proceeding direction) and are arranged so as to be spaced apart from each other in the direction (left-right direction) orthogonal to the front-rear direction. Each of the transverse frame members 82 extends in the left-right direction and is connected to the longitudinal frame members 81. A pair of combinational structures each including the longitudinal frame members 81 and the transverse frame members 82 are provided in the upper-lower direction. The base mount 80 further includes a plurality of vertical frame members 83 each connected to the longitudinal frame member at an upper side and the longitudinal frame member 81 at a lower side. It should be noted that in FIG. 5, the casing 71 is not shown for explanation of the base mount 80.

A plate-shaped base plate 84 is provided on the two transverse frame members 82 arranged at the upper side of the base mount 80. The base 20 holding the positioner 7 is attached to the base plate 84. It should be noted that the base plate 84 is configured so as to be covered with the casing constituting portion 71a.

A frame member 87 having, for example, a U shape (substantially U shape) in plan view is connected to a pair of vertical frame members 83 arranged at the front side. A frame member 85 extending in the left direction is connected to one of end portions of the frame member 87, and a frame member 86 extending in the right direction is connected to the other end portion of the frame member 87. A pair of pneumatic front stabilizers 11 and 12 configured to be able to be extended and contracted are provided at the frame members 85 and 86, respectively. The front stabilizers 11 and 12 are arranged so as to be spaced apart from each other in the direction orthogonal to the proceeding direction. Moreover, the front stabilizers 11 and 12 are arranged between the right front wheel 40 and the left front wheel 41 in the direction orthogonal to the proceeding direction.

The front stabilizer 11 includes: an air cylinder 11a; a piston rod 11b provided so as to advance or retreat relative to the air cylinder 11a by pneumatic pressure; and a ground contact portion 11c provided at a tip end side of the piston rod 11b and having, for example, a circular plate shape. Similarly, the front stabilizer 12 includes: an air cylinder 12a; a piston rod 12b provided so as to be able to advance or retreat relative to the air cylinder 12a by pneumatic pressure; and a ground contact portion 12c provided at a tip end side of the piston rod 12b and having, for example, a circular plate shape. Moreover, in the present embodiment, at least part of the ground contact portion 11c of the front stabilizer 11 and at least part of the ground contact portion 12c of the front stabilizer 12 are arranged at the front side of a frontmost portion of the right front wheel 40 and a frontmost portion of the left front wheel 41 in the proceeding direction.

Moreover, a pair of pneumatic rear stabilizers 13 and 14 configured to be able to be extended and contracted are respectively provided at the right and left vertical frame members 83 located at the rear side of the base mount 80. The rear stabilizers 13 and 14 are arranged so as to be spaced apart from each other in the direction orthogonal to the proceeding direction. Moreover, the rear stabilizer 13 is arranged outside the right rear wheel 43 in the above orthogonal direction, and the rear stabilizer 14 is arranged outside the left rear wheel 44 in the above orthogonal direction. To be specific, the rear stabilizers 13 and 14 are arranged so as to sandwich the right rear wheel 43 and the left rear wheel 44 in the above orthogonal direction. It should be noted that the frame structure of the base mount 80 is not limited to the structure shown in FIG. 5. Moreover, the front stabilizers 11 and 12 and the rear stabilizers 13 and 14 are only required to be provided at the base mount 80, and arrangement positions of the stabilizers 11 to 14 (arrangement positions of the stabilizers 11 to 14 at the base mount 80) are not limited to those shown in FIG. 5.

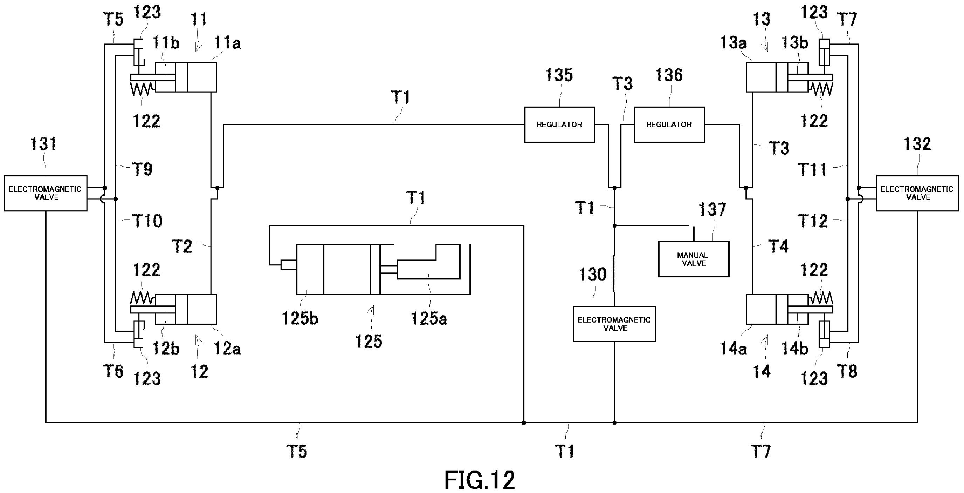

As with the front stabilizers 11 and 12, the rear stabilizer 13 includes: an air cylinder 13a (FIG. 12); a piston rod 13b (FIG. 12) provided so as to be able to advance or retreat relative to the air cylinder 13a by pneumatic pressure; and a ground contact portion 13c provided at a tip end side of the piston rod 13b and having, for example, a circular plate shape, and the rear stabilizer 14 includes: an air cylinder 14a (FIG. 12); a piston rod 14b (FIG. 12) provided so as to be able to advance or retreat relative to the air cylinder 14a by pneumatic pressure; and a ground contact portion 14c provided at a tip end side of the piston rod 14b and having, for example, a circular plate shape.

According to this configuration, when the medical cart 70 moves, the piston rods 11b, 12b, 13b, and 14b contract such that the ground contact portions 11c, 12c, 13c, and 14c float from a ground surface Gr (see FIG. 7). With this, the movement of the medical cart 70 is not inhibited.

On the other hand, when stopping the medical cart 70 such that the medical cart 70 does not move from a desired position after the medical cart 70 is moved to the desired position, as shown in FIG. 7, the piston rods 11b, 12b, 13b, and 14b are extended such that the ground contact portions 11c, 12c, 13c, and 14c contact the ground surface Gr. In this case, the ground contact portions 11c, 12c, 13c, and 14c contact the ground surface Gr in a state where the right front wheel 40, the left front wheel 41, the right rear wheel 43, and the left rear wheel 44 do not float from the ground surface Gr (i.e., in a state where the wheels are in contact with the ground surface Gr).

Next, as shown in FIG. 6, as components of a control system of the medical cart 70 of the present embodiment, the medical cart 70 includes the positioner control portion 603, brake devices 26, electromagnetic valves 130, 131, and 132, and a compressed air supply source 125 in addition to the steering portion 48, the input device 106, the driving servomotors M40a and M41a, and the stabilizers 11, 12, 13, and 14. The brake devices 26 are provided so as to respectively correspond to the right front wheel 40 and the left front wheel 41 and respectively brake and lock the right front wheel 40 and the left front wheel 41. It should be noted that the electromagnetic valves 130, 131, and 132 and the compressed air supply source 125 will be described later.

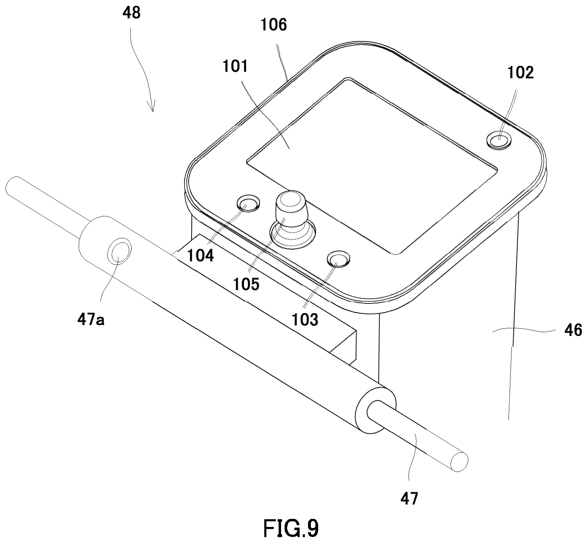

As shown in FIG. 9, the input device 106 includes a touch panel display 101, an emergency stop switch 102, a power supply switch 103, a trigger switch 104, and a joystick 105.

The touch panel display 101 includes a panel-shaped display unit and a contact-type input unit. When the medical worker presses a mark, such as a button, displayed on the touch panel display 41, the touch panel display 41 receives an input of manipulation corresponding to the mark. Information of the input of the manipulation received by the touch panel display 41 is transmitted to the positioner control portion 603.

Moreover, the medical worker can press a predetermined button on the touch panel display 41 to give instructions of ground contact operations or ground contact releasing operations of the stabilizers 11, 12, 13, and 14 to the positioner control portion 603. In this case, the input device 106 receives the instructions of the ground contact operations or the ground contact releasing operations of the stabilizers 11, 12, 13, and 14 from the medical worker and transmits the instructions to the positioner control portion 603. The positioner control portion 603 controls extending operations of the stabilizers 11, 12, 13, and 14 such that the ground contact portions 11c, 12c, 13c, and 14c contact the ground surface Gr when the medical cart 70 is in a stop state. As above, in the present embodiment, the medical worker can instruct the extending operations or the contracting operations (the ground contact operations or the ground contact releasing operations) of the stabilizers 11, 12, 13, and 14 by using the input device 106.

A contact point of the emergency stop switch 102 is of a normally closed type. When the emergency stop switch 102 is not being pressed, the contact point is in a closed state, and a current indicating a safe state flows. When the the emergency stop switch 102 is continuously pressed for a predetermined period of time (for example, several seconds), the contact point opens, and the current is cut off. When the emergency stop switch 102 is pressed during the use of the medical manipulator 1, an instruction of emergency stop is transmitted to the controller 600. The controller 600 acquires the instruction of emergency stop and stops the operation of the medical manipulator 1.

The power supply switch 103 can be turned on or off by being continuously pressed for a predetermined period of time (for example, several seconds). When the power supply switch 103 is turned on, electric power is supplied to the input device 106, and the input device 106 starts up. Moreover, when the power supply switch 103 is turned off, the supply of the electric power to the input device 106 is stopped, and the input device 106 stops.

The trigger switch 104 is a so-called deadman switch. The trigger switch 104 becomes an on state only while the medical worker is pressing the trigger switch 104. The trigger switch 104 becomes an off state when the pressing force is eliminated. Only while the trigger switch 104 is in the on state, the manipulation of the joystick 105 is valid. While the trigger switch 104 is in the off state, the manipulation of the joystick 105 is invalid. The controller 600 detects whether the trigger switch 104 is in the on state or the off state. It should be noted that the trigger switch 104 may be a three-position enable switch.

The joystick 105 is an operation tool which receives an input of manipulation information regarding an operation amount and a movement direction. In the present embodiment, the joystick 105 has a knob shape or a lever shape which can tilt in front, rear, left, and right directions and rotate forward and backward. A tilting direction of the joystick 105 corresponds to the input movement direction. A rotational direction of the joystick 105 corresponds to an input rotational direction. Each of t tilting time and a rotating time from a zero position of the joystick 105 corresponds to the input operation amount. Information of the input of the manipulation received by the joystick 105 is transmitted to the controller 600. Only while the trigger switch 104 is in the on state, the controller 600 receives an input from the joystick 105. Various input units provided at the input device 106 acquire an input of received manipulation and transmit information corresponding to the input manipulation to the controller 600. The controller 600 operates the medical manipulator 1 based on information acquired from the input device 106.

As shown in FIG. 9, the handle 47 is provided with a release switch 47a as a brake releasing tool configured to release the braking of the brake devices 26 in an emergency. When the medical worker presses down the release switch 47a, a current flows through the brake devices 26 that are, for example, non-excitation operation type electromagnetic brakes, and the braking of the brake devices 26 is released. When the medical cart 70 is in a stop state, the braking of the brake devices 26 works at all times. When manipulating the handle 47, the medical worker presses down the release switch 47a to release the braking of the brake devices 26. Hereinafter, the configuration of realizing the braking of the brake devices 26 and the release of the braking will be described based on one example.

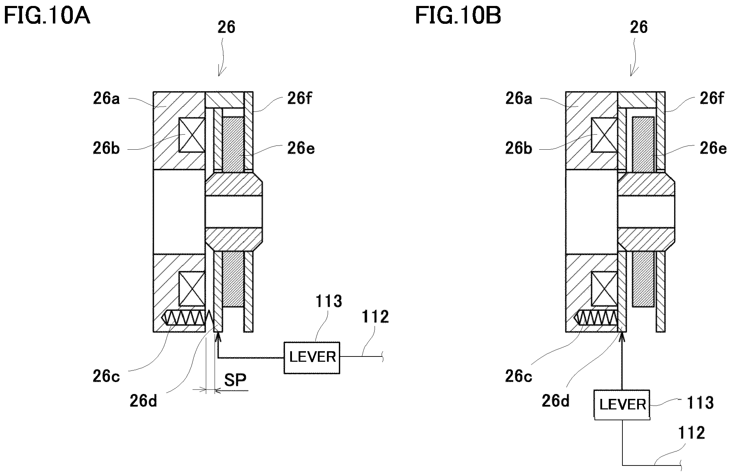

As described above, the brake devices 26 are constituted by, for example, the non-excitation operation type electromagnetic brakes. It should be noted that the brake devices 26 are not limited to the non-excitation operation type electromagnetic brakes, and the other brake devices may be adopted. Since the non-excitation operation type electromagnetic brake is publicly known, an explanation thereof will be briefly made below. As shown in FIGS. 10A and 10B, each of the brake devices 26 includes a stator 26a, a coil 26b, a torque spring 26c, an armature 26d, a rotor 26e coupled to an output shaft (front wheel rotating shaft) of the driving servomotor M40a or M41a, and a plate 26f.

According to this configuration, when a current is not supplied to the coil 26b, as shown in FIG. 10A, the armature 26d is biased by the torque spring 26c in a right direction in FIG. 10A, and with this, the rotor 26e is sandwiched between the plate 26f and the armature 26d. Thus, the output shaft of the driving servomotor M40a or M41a is restricted so as to be unrotatable. In this case, a space SP is formed between the stator 26a and the armature 26d. In contrast, when a current is supplied to the coil 26b, as shown in FIG. 10B, magnetic force is generated at the coil 26b, and the armature 26d is attracted toward the stator 26a by the magnetic force against the biasing force of the torque spring 26c. In this case, the space SP between the stator 26a and the armature 26d disappears. Therefore, the rotor 26e is not sandwiched between the plate 26f and the armature 26d and becomes a free state. With this, the braking of the rotor 26e is released, and the rotor 26e can perform a rotating operation. When the medical worker presses down the release switch 47a, a current flows through the coil 26b, and the braking of the brake devices 26 can be released as explained above with reference to FIG. 10B.

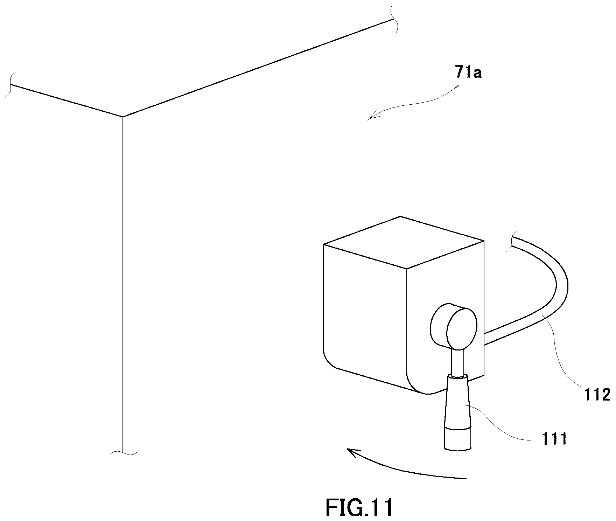

As shown in FIG. 11, an operating lever 111 as a manual operation tool is provided on a rear surface (back surface) of the casing constituting portion 71a. The operating lever 111 is used to operate a wire 112, a lever 113 (see FIG. 10), and a below-described manual valve 137 . The wire 112 and the lever 113 serve as a brake releasing tool configured to release the braking of the brake devices 26 that are the electromagnetic brakes. Details will be described below.

For example, the operating lever 111 is configured to be rotatable clockwise and counterclockwise. One of ends of the wire 112 is connected to the operating lever 111. The other end of the wire 112 is connected to the lever 113 shown in FIGS. 10A and 10B. The lever 113 is attached to the armatures 26d of the brake devices 26. According to this configuration, when the medical worker rotates the operating lever 111, for example, clockwise in an emergency, the wire 112 is pulled by this rotation, and the armatures 26d can be moved toward the stators 26a through the lever 113 which operates in accordance with the movement of the wire 112. Thus, when power supply is cut off (in an emergency), the medical worker can manually release the braking of the brake devices 26 by using the operating lever 111 (see FIG. 10B).

Moreover, in the present embodiment, by rotating the operating lever 111 as above, the ground contact releasing operations of the stabilizers 11, 12, 13, and 14 can be manually performed. Details will be described later.

FIG. 12 is a diagram showing a pneumatic circuit which realizes the expansion and contraction of the stabilizers 11, 12, 13, and 14. As shown in FIG. 12, the stabilizer 11 (12, 13, 14) includes: the air cylinder 11a (12a, 13a, 14a) including the piston rod 11b (12b, 13b, 14b) which can advance or retreat; a return spring 122; and the ground contact portion 11c (12c, 13c, 14c) provided at a tip end portion of the piston rod 11b (12b, 13b, 14b) (see FIG. 8).

The compressed air supply source 125 configured to supply compressed air to the air cylinders 11a, 12a, 13a, and 14a is provided at the medical cart 70. The compressed air supply source 125 is, for example, an electric air booster and includes an electric cylinder 125a and a sub-tank 125b. The air cylinder 11a of the stabilizer 11 and the compressed air supply source 125 are connected to each other through an air passage T1, and the air cylinder 12a of the stabilizer 12 and the compressed air supply source 125 are connected to each other through an air passage T2 branching from the air passage T1. Moreover, the air cylinder 13a of the stabilizer 13 and the compressed air supply source 125 are connected to each other through an air passage T3 branching from the air passage T1, and the air cylinder 14a of the stabilizer 14 and the compressed air supply source 125 are connected to each other through an air passage T4 branching from the air passage T3.

A three-position electromagnetic valve 130 is interposed on a portion of the air passage T1 which portion is located upstream of a branch point where the air passage T3 branches from the air passage T1. Moreover, a regulator 135 is interposed on a portion of the air passage T1 which portion is located between the electromagnetic valve 130 and a branch point where the air passage T2 branches from the air passage T1. Similarly, a regulator 136 is interposed on a portion of the air passage T3 which portion is located upstream of a branch point where the air passage T4 branches from the air passage T3. Furthermore, a two-position manual valve 137 is interposed on a portion of the air passage T1 which portion is located between the electromagnetic valve 130 and the branch point where the air passage T3 branches from the air passage T1. It should be noted that the regulator 135 can adjust the pressure of the air to be supplied to the air cylinder 1 1a of the stabilizer 11 and the air cylinder 12a of the stabilizer 12, and the regulator 136 can adjust the pressure of the air to be supplied to the air cylinder 13a of the stabilizer 13 and the air cylinder 14a of the stabilizer 14.

An air passage T5 branches from a portion of the air passage T1 which portion is located upstream of the electromagnetic valve 130. An opposite end of the air passage T5 is connected to a lock cylinder 123, and a piston rod (not shown) is driven by pneumatic pressure to lock the piston rod 11b of the stabilizer 11. A two-position electromagnetic valve 131 is interposed on the air passage T5. Moreover, an air passage T6 branches from a portion of the air passage T5 which portion is located downstream of the electromagnetic valve 131. An opposite end of the air passage T6 is connected to a lock cylinder 123 in order to lock the piston rod 12b of the stabilizer 12. Furthermore, an air passage T9 is provided so as to connect the electromagnetic valve 130 and an opposite end of the lock cylinder 123 of the stabilizer 11. An air passage T10 branches from the air passage T9 and includes an opposite end connected to an opposite end of the lock cylinder 123 of the stabilizer 12. Similarly, an air passage T7 branches from a portion of the air passage T1 which portion is located upstream of the electromagnetic valve 130. An opposite end of the air passage T7 is connected to a lock cylinder 123 in order to lock the piston rod 13b of the stabilizer 13. A two-position electromagnetic valve 132 is interposed on the air passage T7. Moreover, an air passage T8 branches from a portion of the air passage T7 which portion is located downstream of the electromagnetic valve 132. An opposite end of the air passage T8 is connected to a lock cylinder 123 in order to lock the piston rod 14b of the stabilizer 14. Furthermore, an air passage T11 is provided so as to connect the electromagnetic valve 132 and an opposite end of the lock cylinder 123 of the stabilizer 13. An air passage T12 branches from the air passage T11 and includes an opposite end connected to an opposite end of the lock cylinder 123 of the stabilizer 14.

According to the above configuration, when the medical worker manipulates the input device 106 to instruct the ground contact operation, one of solenoid coils of the electromagnetic valve 130 is energized, and with this, a valve element of the electromagnetic valve 130 moves to open a supply passage. With this, the compressed air from the compressed air supply source 125 is supplied to the air passages T1, T2, T3, and T4, and therefore, the piston rods 11b, 12b, 13b, and 14b are extended. Thus, the ground contact portions 11c, 12c, 13c, and 14c contact the ground surface, and then, the solenoid coil of the electromagnetic valve 130 is set to a non-energized state. At this time, the supply passage of the electromagnetic valve 130 is closed. In addition, when solenoid coils of the electromagnetic valves 131 and 132 are energized, valve elements of the electromagnetic valves 131 and 132 move to open supply passages. Thus, the compressed air is supplied to the air passages T5, T6, T7, and T8. With this, the lock cylinders 123 of the stabilizers 11, 12, 13, and 14 operate, and the extended states of the piston rods 11b, 12b, 13b, and 14b are held (locked). Thus, the air cylinders 11a, 12a, 13a, and 14a are constituted as pneumatic lock air cylinders. On the other hand, when the medical worker manipulates the input device 106 to instruct the ground contact releasing operation, the solenoid coils of the electromagnetic valves 131 and 132 are first set to a non-energized state, and with this, the valve elements of the electromagnetic valves 131 and 132 move to open discharge passages of the electromagnetic valves 131 and 132. Thus, the operating states of the lock cylinders 123 are released, and therefore, the piston rods 11b, 12b, 13b, and 14b are unlocked. Next, when the other solenoid coil of the electromagnetic valve 130 is energized, the valve element of the electromagnetic valve 130 moves to open a discharge passage of the electromagnetic valve 130. With this, the compressed air in the air cylinders 11a, 12a, 13a, and 14a is discharged, and therefore, the piston rods 11b, 12b, 13b, and 14b contract. Thus, the ground contact states of the ground contact portions 11c, 12c, 13c, and 14c are released. Then, the other solenoid coil of the electromagnetic valve 130 is set to a non-energized state. By the above method, the ground contact operations and the ground contact releasing operations of the ground contact portions of the stabilizers are performed.

In a case where the medical worker manually performs the ground contact releasing operations of the stabilizers when power supply is cut off, the medical worker rotates the operating lever 111 clockwise as described above. With this, a valve element of the manual valve 137 coupled to the operating lever 111 through a pin and the like can move to open a discharge passage of the manual valve 137. With this, the compressed air in the air cylinders 11a, 12a, 13a, and 14a is discharged, and therefore, the piston rods 11b, 12b, 13b, and 14b are biased by the return springs 122. Thus, the piston rods 11b, 12b, 13b, and 14b contract. With this, the ground contact states of the ground contact portions 11c, 12c, 13c, and 14c are released. As above, the medical worker can manually release the ground contact states of the stabilizers when power supply is cut off.

As described above, according to the medical cart 70 of the present embodiment, when the wheels are in a stop state, the ground contact portions 11c, 12c, 13c, and 14c contact the ground surface Gr by the expansion of the stabilizers 11, 12, 13, and 14. Therefore, the medical cart 70 can be fixed at a stop position. With this, the medical cart 70 can be prevented from moving to an unexpected position. Especially, since the medical cart 70 is prevented from moving to an unexpected position during surgery, the medical cart 70 is advantageous in that there is no adverse influence on the surgery. Moreover, since the stabilizers 11, 12, 13, and 14 are extended or contract by pneumatic pressure, the stabilizers 11, 12, 13, and 14 are more sanitary than stabilizers configured to be extended or contracted by hydraulic pressure, and therefore, are preferably used in surgery sites.

Moreover, in the present embodiment, the ground contact portions 11c, 12c, 13c, and 14c of the stabilizers 11, 12, 13, and 14 contact the ground surface Gr in a state where the wheels do not float from the ground surface Gr. With this, when removing air from the stabilizers 11, 12, 13, and 14, i.e., when making the stabilizers 11, 12, 13, and 14 contract, impact applied to the casing 71 since the wheels are not in contact with the ground surface can be prevented.

Moreover, in the present embodiment, since the front stabilizers 11 and 12 are arranged between the right front wheel 40 and the left front wheel 41 in the direction orthogonal to the proceeding direction, the front stabilizers 11 and 12 do not interfere with the rotating operations of the right front wheel 40 and the left front wheel 41 as compared to when the front stabilizer 11 is arranged outside the right front wheel 40 in the same direction, and the front stabilizer 12 is arranged outside the left front wheel 41 in the same direction.

Moreover, in the present embodiment, the rear stabilizer 13 is arranged outside the right rear wheel 43 in the direction orthogonal to the proceeding direction, and the rear stabilizer 14 is arranged outside the left rear wheel 44 in the same direction. With this, the small interval between the right rear wheel 43 and the left rear wheel 44 can be designed. Thus, the medical worker can change the proceeding direction of the medical cart 70 by small force.

Furthermore, in the present embodiment, in a state where the wheels are stopped by the braking of the brake devices 26, the ground contact portions 11c, 12c, 13c, and 14c of the stabilizers 11, 12, 13, and 14 contact the ground surface Gr. Therefore, the medical cart 70 can be surely stopped so as not to move.

Moreover, in the present embodiment, since the interval between the right rear wheel 43 and the left rear wheel 44 is set to be smaller than an interval between the right front wheel 40 and the left front wheel 41, operability when changing the directions of the rear wheels 43 and 44 by the handle 47 can be improved.

Moreover, in the present embodiment, the interval between the pair of auxiliary wheels 110 is larger than the interval between the right rear wheel 43 and the left rear wheel 44. Therefore, the directions of the rear wheels 43 and 44 can be easily changed by the existence of the auxiliary wheels 110.

Moreover, in the present embodiment, in an emergency which requires the release of the braking of the brake devices 26, such release can be easily and quickly performed by the wire 112 and the lever 113 which serve as the brake releasing tool.

Moreover, in the present embodiment, the piston rods 11b, 12b, 13b, and 14b can be easily extended by the air cylinders 11a, 12a, 13a, and 14a, and with this, the ground contact portions 11c, 12c, 13c, and 14c can be made to contact the ground surface.

Moreover, in the present embodiment, since the air cylinders 11a, 12a, 13a, and 14a are the pneumatic lock air cylinders, the extended states of the piston rods 11b, 12b, 13b, and 14b of the air cylinders 11a, 12a, 13a, and 14a can be locked by using pneumatic pressure. Thus, the extended states of the stabilizers 11, 12, 13, and 14 can be held (locked).

Moreover, in the present embodiment, when releasing the air in the air cylinders 11a, 12a, 13a, and 14a of the stabilizers 11, 12, 13, and 14, the piston rods 11b, 12b, 13b, and 14b can be made to contract by the biasing force of the return springs 122, and therefore, the ground contact states of the stabilizers 11, 12, 13, and 14 can be released.

Moreover, in the present embodiment, the compressed air can be supplied to four air cylinders 11a, 12a, 13a, and 14a by one compressed air supply source 125.

Moreover, in the present embodiment, in an emergency, such as when power supply is cut off, the air in the air cylinders 11a, 12a, 13a, and 14a of the stabilizers 11, 12, 13, and 14 can be released by the manual valve 137. With this, the stabilizers 11, 12, 13, and 14 can be made to contract by a manual method, and the ground contact states of the stabilizers 11, 12, 13, and 14 can be released. Thus, the medical cart 70 can be set to a movable state.

Moreover, in the present embodiment, in an emergency, such when power supply is cut off, both the brake releasing tool (the wire 112 and the lever 113) and the manual valve 137 can be easily operated by the operating lever 111 as the manual operation tool. With this, the braking of the brake devices 26 can be released, and the ground contact states of the stabilizers 11, 12, 13, and 14 can also be released.

Furthermore, in the present embodiment, the medical worker can easily instruct the ground contact operations and the ground contact releasing operations of the stabilizers 11, 12, 13, and 14 by using the input device 106.

### Other Embodiments

In addition to the above embodiment, various modifications below may be made within the scope of the present invention.

In the above embodiment, the front stabilizers 11 and 12 are provided as two stabilizers at the front side of the base mount 80, and the rear stabilizers 13 and 14 are provided as two stabilizers at the rear side of the base mount 80. However, the above embodiment is not limited to this. Three or more stabilizers may be provided at the front side of the base mount 80, and three or more stabilizers may be provided at the rear side of the base mount 80.

Moreover, in the above embodiment, the number of rear wheels is two. However, the above embodiment is not limited to this, and the number of rear wheels may be set to one by, for example, increasing the width of the rear wheel.

Moreover, in the above embodiment, at least part of the ground contact portion 11c of the stabilizer 11 and at least part of the ground contact portion 12c of the stabilizer 12 are arranged at the front side of the frontmost portion of the right front wheel 40 and the frontmost portion of the left front wheel 41 in the proceeding direction. However, the above embodiment is not limited to this. The entire ground contact portion 11c and the entire ground contact portion 12c may be arranged between the frontmost portion and rearmost portion of the right front wheel 40 in the proceeding direction and between the frontmost portion and rearmost portion of the left front wheel 41 in the proceeding direction. Or, part of the ground contact portion 11c and part of the ground contact portion 12c may be arranged at the rear side of the rearmost portion of the right front wheel 40 and the rearmost portion of the left front wheel 41 in the proceeding direction.

Furthermore, in the above embodiment, the pneumatic lock air cylinders are used as the air cylinders. However, the above embodiment is not limited to this. Spring lock air cylinders or spring-pneumatic pressure combination lock air cylinders may be used.

### Reference Signs List

- 1: medical manipulator
- 3: multiple degree-of-freedom manipulator arm
- 4: medical device
- 7: positioner
- 11, 12: front stabilizer
- 11a, 12a, 13a, 14a: air cylinder
- 11b, 12b, 13b, 14b: piston rod
- 11c, 12c, 13c, 14c: ground contact portion
- 13, 14: rear stabilizer
- 20: base
- 26: brake device
- 40: right front wheel
- 41: left front wheel
- 43: right rear wheel
- 44: left rear wheel
- 46: steering shaft
- 47: handle
- 47a: release switch
- 48: steering portion
- 70: medical cart
- 80: base mount
- 106: input device
- 110: auxiliary wheel
- 111: operating lever (manual operation tool)
- 112: wire (brake releasing tool)
- 113: lever (brake releasing tool)
- 122: return spring (biasing member)
- 125: compressed air supply source
- 130, 131, 132: electromagnetic valve
- 137: manual valve
- Gr: ground surface

## Claims

1. A medical cart comprising:
a base holding a positioner configured to move a multiple degree-of-freedom manipulator arm including a tip end portion holding a medical device;
a base mount holding the base;
a plurality of wheels including a pair of front wheels and a pair of rear wheels which move the base mount; and
a plurality of stabilizers provided at the base mount and including respective ground contact portions which contact a ground surface when the stabilizers are extended by pneumatic pressure.

2. The medical cart according to claim 1, wherein the ground contact portions of the stabilizers contact the ground surface in a state where the wheels do not float from the ground surface.

3. The medical cart according to claim 1 or 2, wherein the plurality of stabilizers comprise two front stabilizer arranged at a front portion of the base mount and two rear stabilizers arranged at a rear portion of the base mount.

4. The medical cart according to claim 3, wherein:
the pair of front wheels comprise a left front wheel and a right front wheel arranged so as to be spaced apart from each other in an orthogonal direction that is a direction orthogonal to a proceeding direction;
the rear wheels comprise a left rear wheel and a right rear wheel arranged so as to be spaced apart from each other in the orthogonal direction;
the two front stabilizers are arranged between the left front wheel and the right front wheel in the orthogonal direction;
one of the rear stabilizers is arranged outside the left rear wheel in the orthogonal direction; and
the other of the rear stabilizers is arranged outside the right rear wheel in the orthogonal direction.

5. The medical cart according to any one of claims 1 to 4, further comprising a steering portion including a steering shaft and a handle provided at the steering shaft, the steering portion being arranged at a rear side of the base mount in a proceeding direction, the steering shaft being used to change directions of the pair of rear wheels, wherein
an interval between the pair of rear wheels is smaller than an interval between the pair of front wheels.

6. The medical cart according to any one of claims 1 to 5, wherein:
the pair of front wheels are driving wheels;
a pair of auxiliary wheels are provided between the pair of front wheels and the pair of rear wheels; and
an interval between the pair of auxiliary wheels is larger than an interval between the pair of rear wheels.

7. The medical cart according to any one of claims 1 to 6, wherein the pair of front wheels are driving wheels,
the medical cart further comprising:
brake devices respectively provided at the pair of front wheels; and
a brake releasing tool configured to release braking of the brake devices in an emergency.

8. The medical cart according to any one of claims 1 to 7, wherein:
each of the plurality of stabilizers includes
an air cylinder including a piston rod configured to advance or retreat and
the ground contact portion provided at a tip end portion of the piston rod.

9. The medical cart according to claim 8, wherein the air cylinders of the plurality of stabilizers are pneumatic lock air cylinders.

10. The medical cart according to clam 8 or 9, wherein each of the plurality of air cylinders includes a biasing member configured to bias the piston rod in such a direction that the piston rod contracts.

11. The medical cart according to any one of claims 8 to 10, further comprising a compressed air supply source configured to supply compressed air to the air cylinders of the plurality of stabilizers.

12. The medical cart according to claim 11, further comprising:
an electromagnetic valve configured to be switchable between a state where the compressed air is supplied from the compressed air supply source to the plurality of air cylinders and a state where the compressed air is not supplied from the compressed air supply source to the plurality of air cylinders; and
a manual valve arranged between the electromagnetic valve and the plurality of air cylinders and configured to discharge the compressed air to an atmosphere.

13. The medical cart according to claim 12, further comprising a manual operation tool configured to operate both the brake releasing tool and the manual valve.

14. The medical cart according to any one of claims 1 to 13, further comprising an input device configured to receive instructions of ground contact operations and ground contact releasing operations of the plurality of stabilizers from an operator.
